# EUROPEAN PATENT APPLICATION

(11) **EP 4 478 039 A1**
(43) Date of publication of application: **18.12.2024**
(21) Application number: 23830811.8
(22) Date of filing: 24.04.2023
(51) Int. Cl.: G01N 27/00, C12M 1/34, G01N 27/28

(54) **SENSOR UNIT, AND CELL CULTURE ANALYZING DEVICE PROVIDED WITH SAME**

(30) Priority: 01.07.2022 JP 2022107106
(71) Applicant: PHC Holdings Corporation, Tokyo 100-8403 (JP)
(72) Inventor: IKETANI, Seiitirou, (JP); YAMAMOTO, Masaki, (JP); NAKAMAE, Kenta, (JP)
(74) Representative: Grünecker Patent- und Rechtsanwälte PartG mbB
(86) International application number: PCT/JP2023/016038
(87) International publication number: WO 2024/004358

(57) **Abstract**

A sensor unit (28) includes a sensor (30), a probe (10a), and a probe holder (10). The probe (10a) makes contact with electrodes (31c) of a connection terminal part (31b) of the sensor (30) and applies a specific voltage. The probe holder (10) holds the probe (10a) so that the probe (10a) protrudes toward the connection terminal part (31b) of the sensor (30), has an opposing surface (61) that is disposed opposite the connection terminal part (31b) of the sensor (30), and forms a non-capillary space (S1) between the opposing surface (61) and the connection terminal part (31b) of the sensor (30) to allow communication with the space facing the adjacent electrodes (31c) of the connection terminal part (31b) of the sensor (30).

## Description

### TECHNICAL FIELD

The present invention relates to a sensor unit for analyzing cultured cells, and to a cell culture analyzing device provided with the same.

### BACKGROUND ART

A conventional cell culture analyzing device is configured such that a measurement device is disposed inside an incubator, a culture module equipped with a sensor and in which cells are cultured is placed in the measurement device, and in this state the progress of the cell culture is measured (the culture status is analyzed) using a glucose sensor while the cells are being cultured.

For instance, Patent Literature 1 discloses a cell culture device including a plurality of culture units, each including a canister that is disposed in a thermostatic bath, and a culture cassette that is housed in the canister.

With the cell culture device disclosed in Patent Literature 1, the culture cassette has a culture bag containing cells to be cultured, and is inserted into the canister. The cells in the culture bag are cultured in an independent culture environment for each canister.

### CITATION LIST

### PATENT LITERATURE

Patent Literature 1: International Patent Application No. WO 2007-052716

### SUMMARY

### TECHNICAL PROBLEM

However, the following problem is encountered with the above-mentioned conventional cell culture device.

With the cell culture device disclosed in the above publication, no consideration whatsoever is given to the occurrence of leakage current attributable to condensation between the measurement electrodes that occurs on the surface of the sensor used in an environment of high temperature and high humidity.

For example, when a sensor that has been placed under a room temperature (25°C) environment has been immersed in a culture medium in a culture container and is then placed in an environment of high heat and humidity in an incubator (37°C, humidity of at least 90%), the temperature of the sensor surface will remain close to room temperature for a time, and is therefore lower than the temperature inside the incubator. Consequently, condensed moisture on the sensor surface may electrically connect the measurement electrodes, resulting in current leakage.

If such a current leak should occur between the measurement electrodes in a cell culture analyzing device that measures minute currents to perform cell culture analysis, etc., there is the risk that various kinds of measurement cannot be performed with high accuracy.

It is an object of the present invention to provide a sensor unit and a cell culture analyzing device provided with the same, with which the occurrence of leakage current attributable to condensation can be effectively suppressed even when the device is used in an environment of high temperature and high humidity.

### SOLUTION TO PROBLEM

The sensor unit according to the first invention is a sensor unit for measuring components of a liquid sample placed in a culture container, including a sensor, a connection part, and a connection part holder. The sensor has a main body, a sensing part that is disposed on the main body and is immersed in the liquid sample, and a connection terminal part including a plurality of electrodes that are electrically connected to the sensing part and to which a specific voltage is applied when the components of the liquid sample are measured. The connection part makes a contact with the electrodes of the connection terminal part of the sensor and applies a specific voltage. The connection part holder holds the connection part so that the connection part protrudes toward the connection terminal part of the sensor, has an opposing surface that is disposed opposite the connection terminal part of the sensor, and forms a non-capillary space between the opposing surface and the connection terminal part of the sensor to allow communication with a space facing the adjacent electrodes of the connection terminal part of the sensor.

### ADVANTAGEOUS EFFECTS

With the sensor unit of the present invention, the occurrence of leakage current due to condensation can be effectively suppressed even when the device used in an environment of high temperature and high humidity.

### BRIEF DESCRIPTION OF DRAWINGS

Fig. 1 is an overall view of the cell culture analyzing device equipped with a sensor unit according to an embodiment of the present invention;
Fig. 2 is an oblique view of the configuration of an analysis unit included in the cell culture analyzing device of Fig. 1;
Fig. 3 is an oblique view showing a state in which a sensor-equipped culture module is placed in the analysis unit of Fig. 2;
Fig. 4 is a schematic diagram showing the processing of a sensor-equipped culture module in a safety cabinet under a room temperature environment;
Figs. 5A and 5B are side views showing a lifting mechanism for lifting a sensor-equipped culture module within an analysis unit;
Fig. 6 is an exploded oblique view of a sensor-equipped culture module including a member that constitutes the lower part of the sensor unit;
Fig. 7 is a top view of a well plate containing a plurality of wells in which the lower ends of the sensors of Fig. 6 are immersed;
Fig. 8 is an oblique view of a plurality of sensors included in the sensor-equipped culture module of Fig. 6;
Fig. 9 is a diagram showing a state in which the lower end of a sensor in Fig. 8 is immersed in a culture medium;
Fig. 10 is a cross-sectional view showing a state in which a sensor in Fig. 8 is sandwiched between a top plate and a bottom plate from above and below;
Fig. 11 is a top view of Fig. 10 as viewed from the top plate side;
Fig. 12 is an oblique view of the configuration of the lower surface side of a probe holder that holds probes whose connection parts make contact with the electrode of the sensor in Fig. 7;
Fig. 13 is an oblique view of the configuration of the upper surface side of the probe holder in Fig. 12;
Fig. 14 is an oblique view showing a state in which the substrate has been removed from the probe holder in Fig. 13;
Fig. 15 is an oblique view showing a state in which the bottom plate and a sensor are placed on a well plate, and the probe holder is set on the top plate;
Fig. 16 is a cross-sectional view showing a state in which the distal ends of the probes make contact with the electrodes of the sensor in the state shown in Fig. 15;
Fig. 17A is a cross-sectional view of a water-repellent resist formed on the electrode surface of the sensor where the electrodes are disposed, and Fig. 17B is a detail cross-sectional view of the A portion in Fig. 17A;
Fig. 18 is a diagram showing an example of the step of forming the water-repellent resist shown in Figs. 17A and 17B;
Figs. 19A to 19C are diagrams showing another example of the steps of forming the water-repellent resist in Fig. 17A and 17B;
Fig. 20A is a cross-sectional view showing a state in which moisture caused by condensation turns into water droplets due to the water-repellent action of the water-repellent resist in Fig. 17A and 17B, so that there is no leakage current between the electrodes, and Fig. 20B is a cross-sectional view of the configuration in a comparative example, and shows a state in which moisture caused by condensation turns into a water film, resulting in leakage current between the electrodes.
Fig. 21A is a cross-sectional view of the configuration around the sensor unit immediately before a used sensor is replaced, and Fig. 21B is a cross-sectional view of the configuration of the sensor unit after the sensor is replaced;
Fig. 22 is an oblique view of the connection part and the connection part holder in a state in which the connection part is facing upward;
Fig. 23 is a cross-sectional view of a structure communicating with the outside, for drying any condensation that occurs around the sensor unit after the sensor is replaced;
Fig. 24A is an oblique view of the configuration of the connection terminal part of a sensor, and Fig. 24B is a cross-sectional view of the connection terminal part of in Fig. 24A along the A-A' line;
Fig. 25A is a detail cross-sectional view of the sensor unit immediately before the sensor is replaced, Fig. 25B is a cross-sectional view showing a state in which the connection part and the electrodes are electrically connected, and Fig. 25C is a detail view of the configuration near the distal end of the connection part; and
Fig. 26 is a graph of the relation between the elapsed time when a disposable sensor is replaced and the temperature of the disposable portion including the sensor.

### DESCRIPTION OF EMBODIMENTS

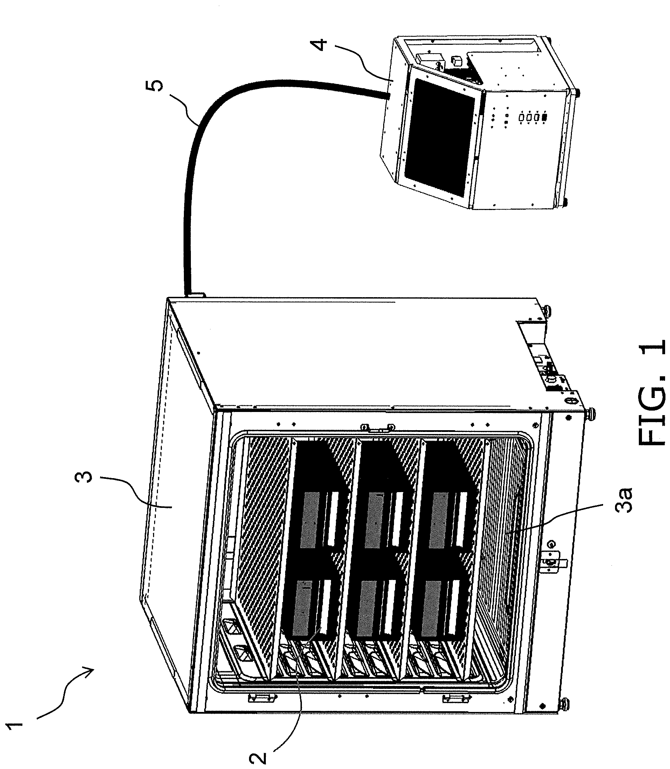

The cell culture analyzing device 1 provided with a sensor unit 28 according to an embodiment of the present invention will now be described with reference to Figs. 1 to 26.

### General Description of Cell Culture Analyzing Device 1

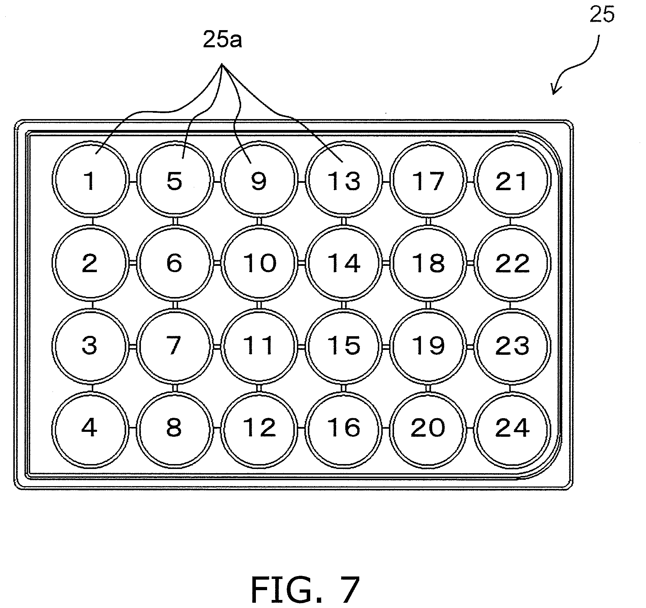

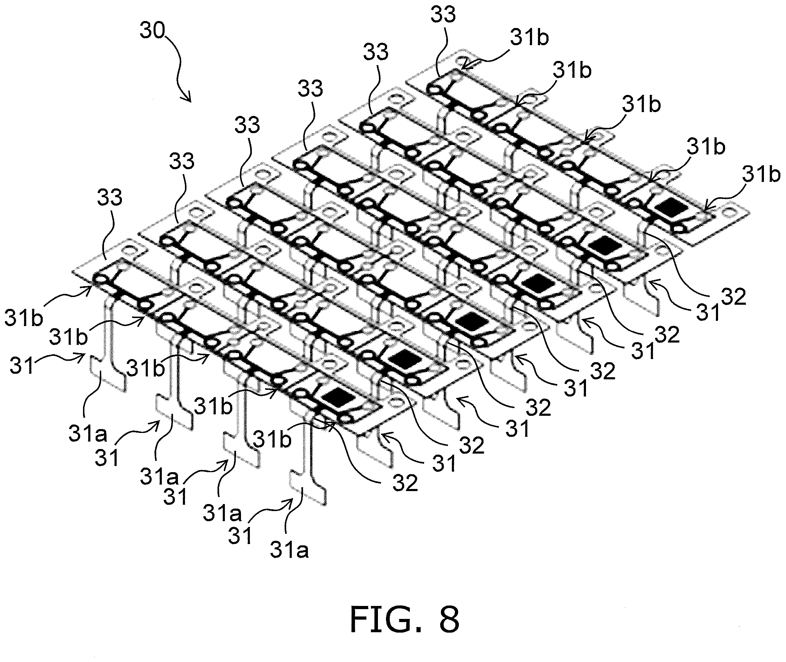

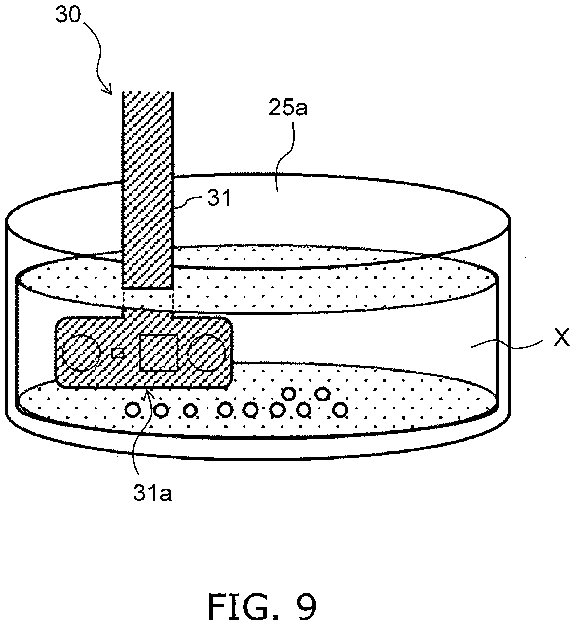

The cell culture analyzing device 1 electrochemically senses the concentration of a specific component (such as glucose or lactic acid) contained in the culture medium (liquid sample) X (see Fig. 9) filling a well plate 25 (see Fig. 7) including a plurality of wells (culture containers) 25a, in a state in which a sensor 30 (see Fig. 8, etc.) is partially immersed in the culture medium X, and analyzes the culture state. As shown in Fig. 1, the cell culture analyzing device 1 includes an analysis unit 2, a culture incubator 3 in whose internal space the analysis unit 2 is placed, and a control unit 4 that controls the analysis unit 2 and displays the analysis result. The analysis unit 2 and the control unit 4 are connected by an electric cable 5.

A transparent door 3a that is attached to the front of the culture incubator 3 is opened and the analysis unit 2 is placed in the internal space. The control unit 4 connected to the analysis unit 2 via the electric cable 5 is disposed outside the culture incubator 3.

This allows the user to analyze the culture state inside the culture incubator 3 with the control unit 4 without having to open and close the door 3a of the culture incubator 3, which prevents contamination of the air inside the culture incubator 3.

The analysis unit 2 is designed to be short in the horizontal (width) direction, low in the height direction, and long in the depth direction so that a plurality of analysis units 2 can be installed in the culture incubator 3.

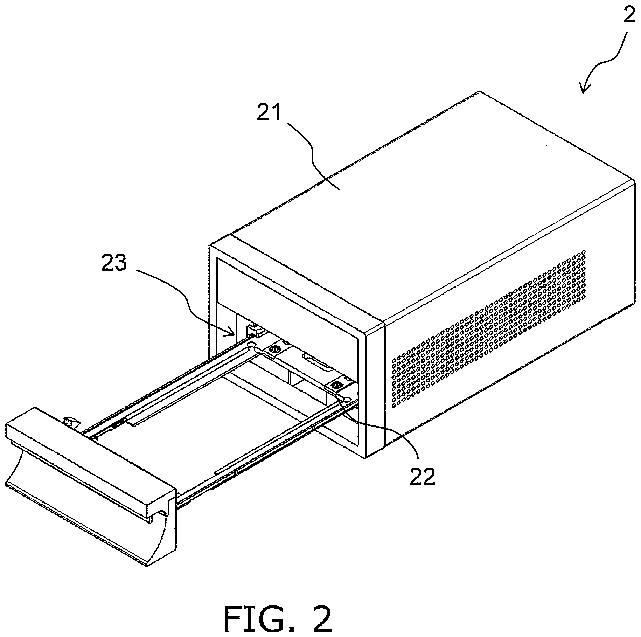

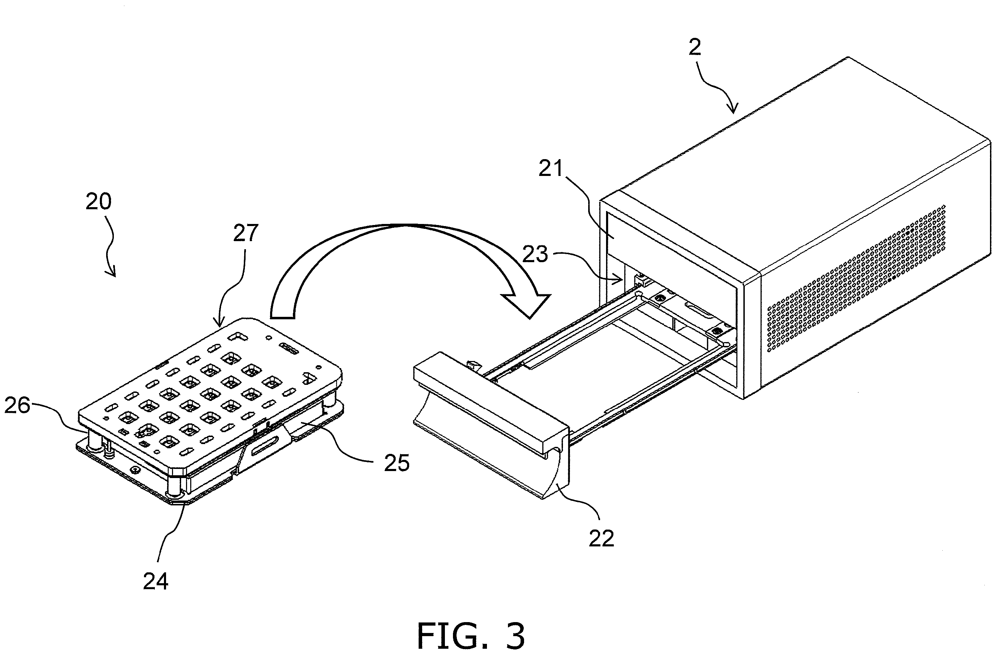

As shown in Figs. 2 and 3, the analysis unit 2 includes a sensor-equipped culture module 20, a main body 21, a drawer 22, and a lifting mechanism 23.

As shown in Fig. 1, the main body 21 is disposed in the culture incubator 3 in advance, and is connected to the control unit 4 by the electric cable 5. During cell culture analysis, the assembled sensor-equipped culture module 20 is placed in the main body 21 inside the culture incubator 3 by the user as shown in Fig. 3.

The analysis unit 2 is configured such that in a state in which the sensor-equipped culture module 20 has been pulled into the main body 21 by the drawer 22, this module can be lifted by the lifting mechanism 23 toward a probe holder (connection part holder) 10 (discussed below).

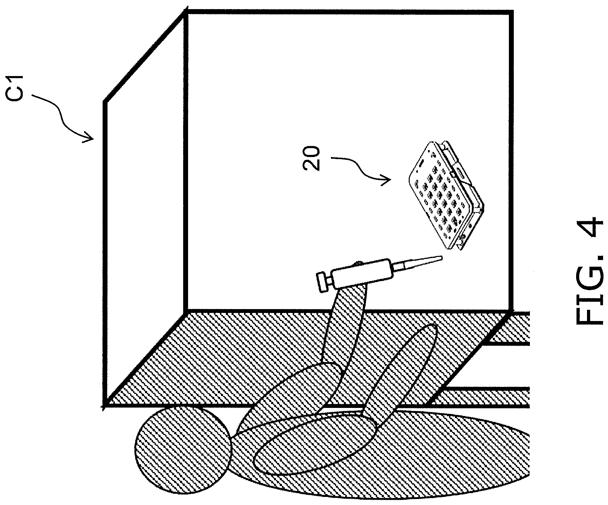

As shown in Fig. 4, the sensor-equipped culture module 20 is assembled inside a safety cabinet C1 under a room temperature environment (such as a temperature of 25°C) after the well plate 25 has been filled with culture medium X (see Fig. 9) and seeded with cells. The assembled sensor-equipped culture module 20 is placed in the analysis unit 2 inside the culture incubator 3, which is held at an environment of high temperature and high humidity (37°C and a humidity of at least 90%). That is, the assembled sensor-equipped culture module 20 is moved from a room temperature environment to an environment of high temperature and high humidity (such as a temperature of 37°C and a humidity of at least 90%).

The configuration for suppressing the occurrence of current leakage attributable to condensation that occurs when the sensor-equipped culture module 20 is moved from a room temperature environment to an environment of high temperature and humidity will be described in detail below.

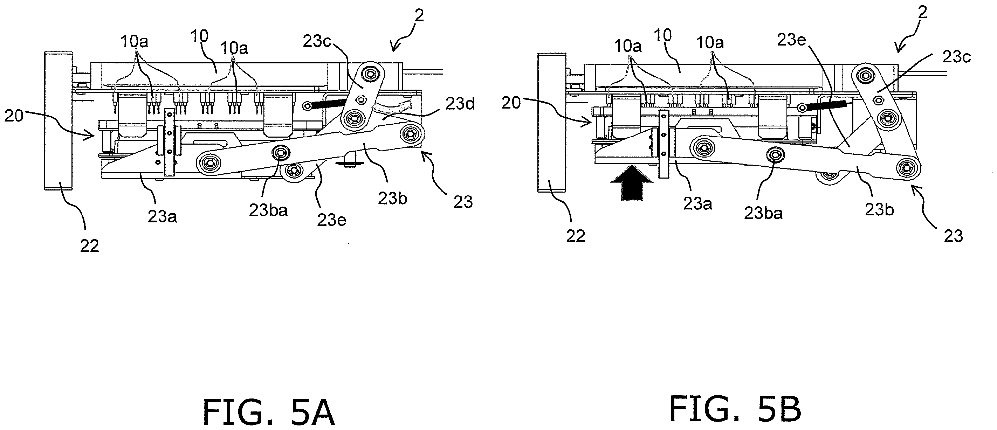

As shown in Figs. 5A and 5B, the lifting mechanism 23 includes a mounting base 23a disposed inside the main body 21, and a link mechanism formed by linking a plurality of arms 23b, 23c, 23d, and 23e.

The sensor-equipped culture module 20 that has been pulled into the internal space of the main body 21 by the drawer 22, which can move from the main body 21 into the external space, is placed on the mounting base 23a, and the mounting base 23a moves up and down when the link mechanism is driven.

More specifically, with the lifting mechanism 23, when the arm 23c is driven counterclockwise in the drawing by a drive unit (not shown), the right end of the arm 23b linked via the arm 23d linked to the other end of the arm 23c, one end of which serves as the rotation center, is pushed down. At this point, the left end of the arm 23b is linked to the side surface of the mounting base 23a.

Consequently, the right end of the arm 23b is pushed down around the rotation center 23ba, and the left end is pushed up, which allows the mounting base 23a on which the sensor-equipped culture module 20 is placed to be lifted upward.

At this point, a probe holder 10 having a probe (connection part) 10a (discussed below) protruding downward is provided in the upper space in which the sensor-equipped culture module 20 has been raised in the analysis unit 2.

This allows the lifting mechanism 23 to lift the sensor-equipped culture module 20 so that the probe 10a comes into contact with the electrode 31c of the sensor 30 included in the sensor-equipped culture module 20.

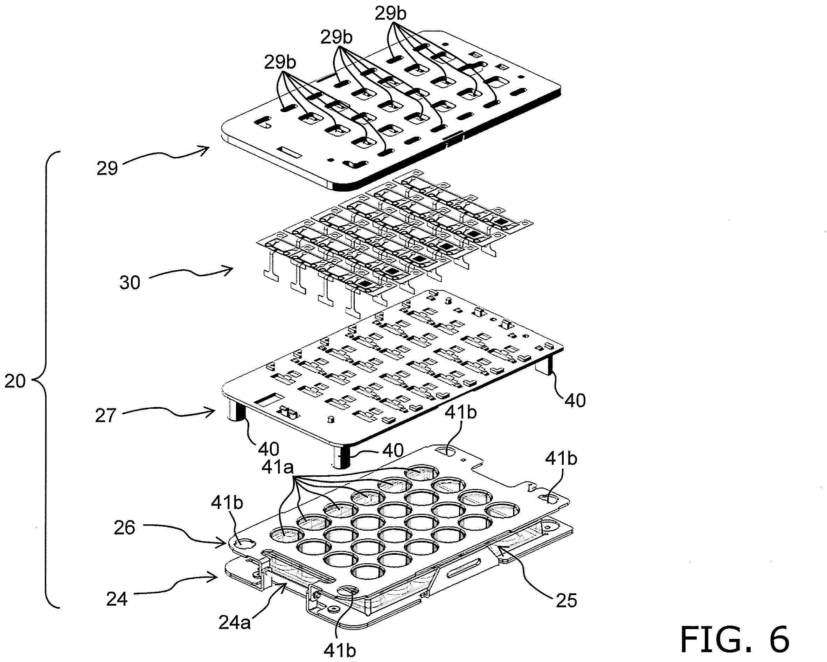

As shown in Fig. 6, the sensor-equipped culture module 20 is configured such that an adaptor bottom 24, a well plate 25, an adaptor top 26, a bottom plate 27, a sensor 30, and a top plate 29 are disposed in that order, starting from the bottom.

The adaptor bottom 24 is connected to the adaptor top 26 via a hinge portion 24a, and the well plate 25 is sandwiched between the adaptor bottom 24 and the adaptor top 26.

As shown in Fig. 7, the well plate 25 is configured to include a total of 24 wells (culture containers) 25a in four vertical rows and six horizontal rows. There are several types of well plate 25, including general-purpose plates, and the adaptor bottom 24 and the adaptor top 26 are selected to suit the type of well plate 25.

As described above, the adaptor top 26 is attached to the adaptor bottom 24 via the hinge portion 24a so as to be openable and closable. The adaptor top 26 has through-holes 41a provided to line up with the positions of the plurality of wells 25a included in the well plate 25, and positioning through-holes 41b provided at the four corners of the flat plate.

The through-holes 41a are provided in four vertical rows and six horizontal rows to line up with the positions of the 24 wells 25a included in the well plate 25. The sensors 30 are immersed in the culture medium X in the wells 25a through the through-holes 41a.

The legs 40 of the bottom plate 27 to which the sensors 30 are attached are inserted into the through-holes 41b, and this positions the sensors 30 relative to the wells 25a.

The bottom plate 27 is disposed over the top surface of the adaptor top 26 in a state in which the plurality of sensors 30 are attached to the top surface (first surface) of the bottom plate 27.

The sensors 30 are configured, for example, by sputtering a carbon electrode layer onto the upper surface of a PET (polyethylene terephthalate) film, which is a resin material. As shown in Fig. 8, the sensors 30 each have a main body 31, a sensing part 31a, a connection terminal part 31b, a bent portion 32, and a linking portion 33.

The main body 31 is a substantially rectangular flat member, and is linked at its upper end portion to the bent portion 32.

As shown in Fig. 9, the sensing part 31a is provided on the surface of a wide portion at the lower end of the downwardly facing, substantially T-shaped main body 31, and includes measurement electrodes (a working electrode, a counter electrode, and a reference electrode). The sensing part 31a is immersed in the culture medium X contained in the well 25a, and a specific voltage is applied to the measurement electrodes, the result being that the concentration of a specific component (such as glucose or lactic acid) contained in the culture medium X is electrochemically measured.

The measurement electrodes included in the sensing part 31a are formed by applying an electrode layer by vapor deposition with a laser and then dividing the electrodes. The measurement electrodes may have an electrode pattern formed by screen printing in order to improve insulation between wirings.

Here, when the concentration of glucose contained in the culture medium X is measured, the reagent layer immobilized on the surface of the working electrode may contain, as a glucose oxidizing enzyme, glucose oxidase (GOx) or glucose dehydrogenase (GDH), as well as a redox mediator, for example.

The glucose concentration is measured as follows. The glucose that has permeated from the culture medium X through the protective membrane is oxidized in a reaction with the enzyme (such as, GOx or GDH) in the reagent layer and becomes gluconolactone, and the electrons generated by the oxidation reaction of hydrogen peroxide or the reduced form of the redox mediator produced at the same time is converted into a current value.

As shown in Fig. 8, the bent portion 32 is the portion that links the main body 31 and the linking portion 33, and is bent at a substantially right angle along a specific bending line. Consequently, the linking portion 33 is disposed at a substantially right angle to the main body 31.

As shown in Fig. 8, the linking portion 33 links the upper ends of the main bodies 31 of the four sensors 30 disposed in the horizontal direction to each other via the bent portions 32.

A connection terminal part 3 1b has four electrodes 31c disposed in a set of four, corresponding to the measurement electrodes of the sensing part 31a of one sensor 30. The four electrodes 31c are electrically connected to the measurement electrodes (working electrode, counter electrode, and reference electrode) included in the sensing part 31a disposed at the lower portion of the main body 31 of the sensor 30.

As shown in Fig. 8, the plurality of sensors 30 included in the sensor unit 28 of this embodiment each include the main body 31, the sensing part 31a that is disposed on the lower end side of the main body 31 and is immersed in the culture medium X to measure a component of the culture medium X, and the linking portion 33 that connects a plurality of sensors 30 to each other on the upper end side of the main body 31.

Consequently, the sensors 30 are attached to the upper surface of the bottom plate 27 in a state of being linked together by the linking portions 33, so the positions of the sensors 30 that are linked together can be accurately determined.

Consequently, the sensors 30 can be positioned (position, angle, etc.) more accurately with respect to the plurality of wells (culture containers) 25a included in the well plate 25.

As a result, the sensors 30 are immersed at a more or less consistent depth in the culture medium X filling the wells 25a, which affords more stable measurement results.

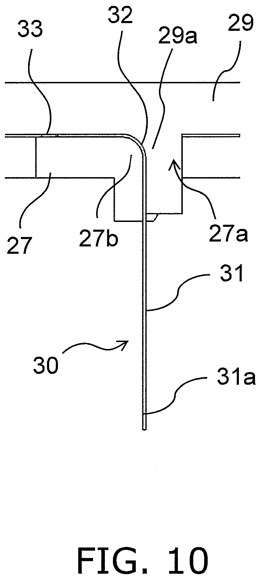

As shown in Figs. 6 and 10, the top plate 29 is disposed so as to cover the upper surface of the sensors 30 attached to the upper surface of the bottom plate 27. The top plate 29 has pressing portions 29a that press the upper side of the bent portions 32 of the sensors 30 downward, and through-holes 29b.

As shown in Fig. 10, the sensors 30 are disposed so as to be sandwiched between the upper surface of the bottom plate 27 and the lower surface of the top plate 29, in a state in which the linking portions 33 are bent.

At this time, each sensor 30 is held between a support portion 27b on the bottom plate 27 side and the pressing portion 29a on the top plate 29 side, as shown in Fig. 10.

That is, the support portion 27b that supports the lower side of the bent portion 32 of the sensor 30 is provided at the opening edge of a through-hole 27a in the bottom plate 27. The pressing portion 29a that presses the upper side of the bent portion 32 of the sensor 30 downward is provided at the portion of the top plate 29 that is opposite the support portion 27b.

Consequently, the upper surface of the sensor 30 is supported by the pressing portion 29a provided on the lower surface side of the top plate 29, and the lower surface of the sensor 30 is supported by the support portion 27b provided on the upper surface side of the bottom plate 27.

The support portion 27b has an upper surface curved portion shape including a curved surface on the upper surface as shown in Fig. 10. The pressing portion 29a has a lower surface curved portion shape including a curved surface on the lower surface as shown in Fig. 10.

Consequently, when the sensor 30 is sandwiched between the top plate 29 and the bottom plate 27 as shown in Fig. 10, the bent portion 32 of the sensor 30 is held in a state of being sandwiched between the support portion 27b and the pressing portion 29a.

The bent angle of the sensor 30 is therefore accurately defined, so the sensing part 31a provided at the lower end of the main body 31 of the sensor 30 is positioned in a stable state. Structure for Suppressing Leakage Current due to Moisture Produced by Condensation

Here, as discussed above, a configuration for suppressing the occurrence of leakage current caused by condensation that occurs when the sensor-equipped culture module 20 is brought from a room temperature environment to an environment of high temperature and humidity will be described.

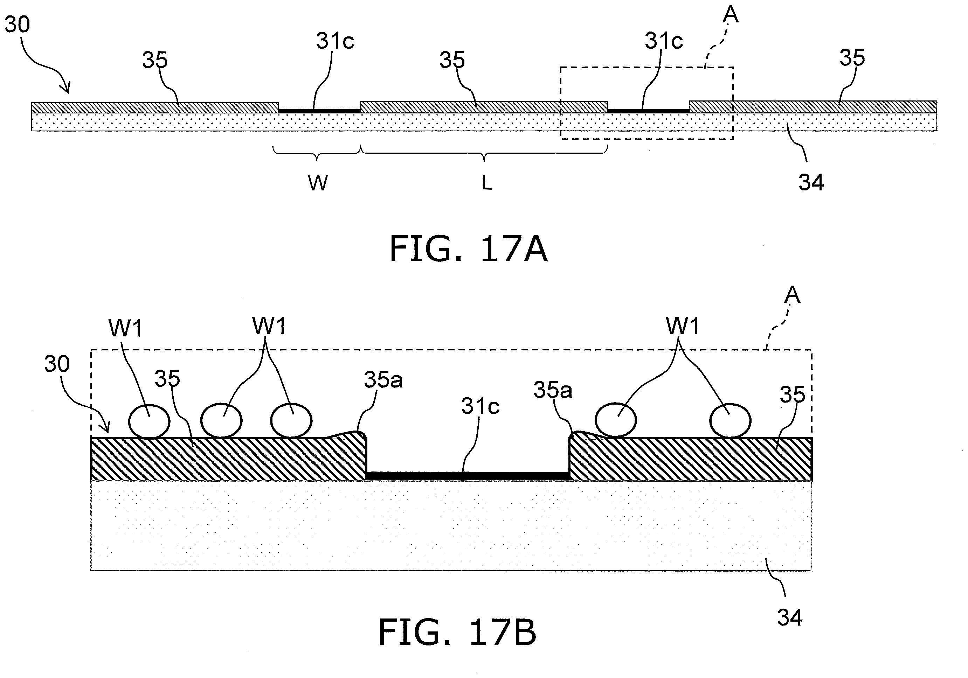

That is, when the sensor-equipped culture module 20 assembled at room temperature (such as 25°C) is placed in the analysis unit 2 inside the culture incubator 3 maintained at an environment of high temperature and high humidity (37°C, humidity of at least 90%), the surface of the sensor 30, which is close to room temperature, is lower than the temperature inside the culture incubator 3, and condensation may occur on the surface of the sensor 30 (see Figs. 17A and 17B). If the electrodes 31c of the sensor 30 are connected by condensation, there is the risk that a current leak will occur, making it impossible to make measurements using a very small current (cell culture analysis) properly.

In view of this, the cell culture analyzing device 1 of this embodiment suppresses the occurrence of leak current with the following configuration.

That is, as shown in Figs. 6 and 8, in the sensor-equipped culture module 20, the top plate 29 has a plurality of the through-holes 29b provided to line up with the positions of the connection terminal parts 31b of the sensors 30, as mentioned above.

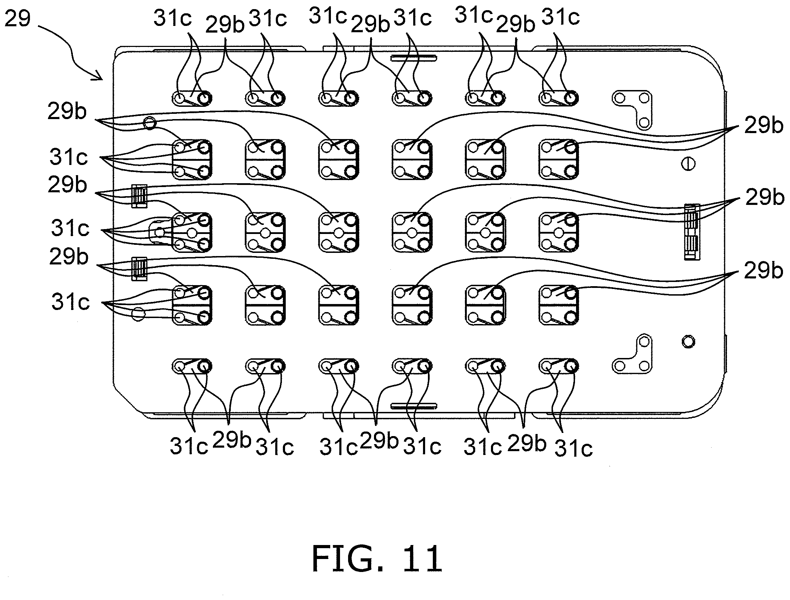

As shown in Fig. 11, the connection terminal parts 31b of the sensors 30 attached to the upper surface of the bottom plate 27 are disposed in the through-holes 29b so as to be exposed when viewed from the upper surface side of the top plate 29. That is, the connection terminal parts 31b are disposed at the bottom portions of the through-holes 29b.

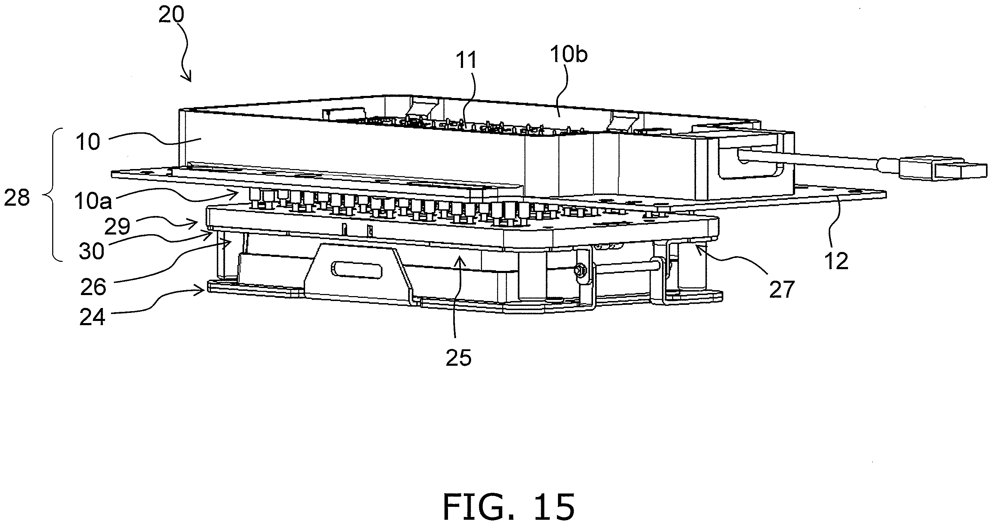

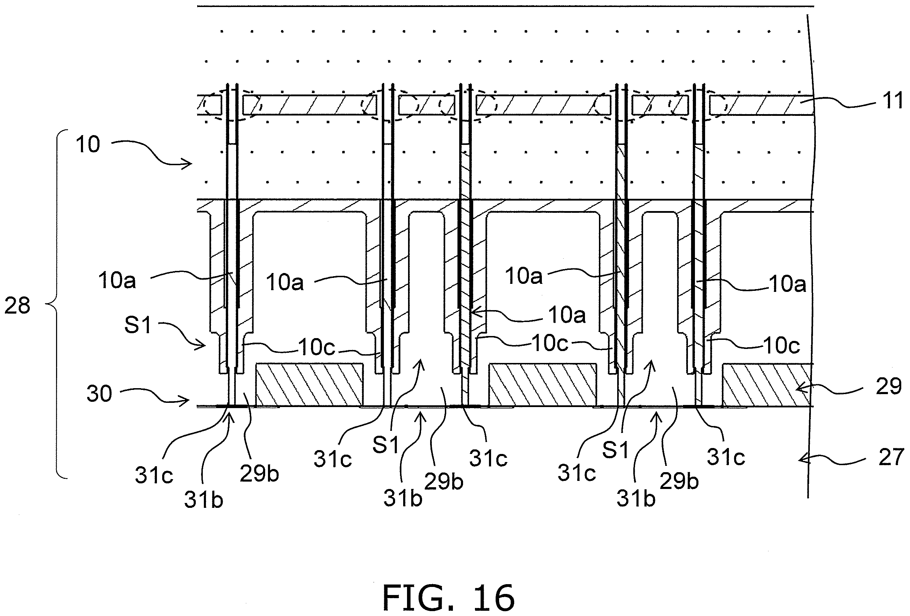

As shown in Figs. 15 and 16, voltage is applied in a state in which the distal ends of the above-mentioned probes 10a are in contact with the electrodes 31c constituting the connection terminal parts 31b via the through-holes 29b in the top plate 29, and the culture medium X is measured by the measurement electrodes electrically connected to the electrodes 31c of the connection terminal parts 31b.

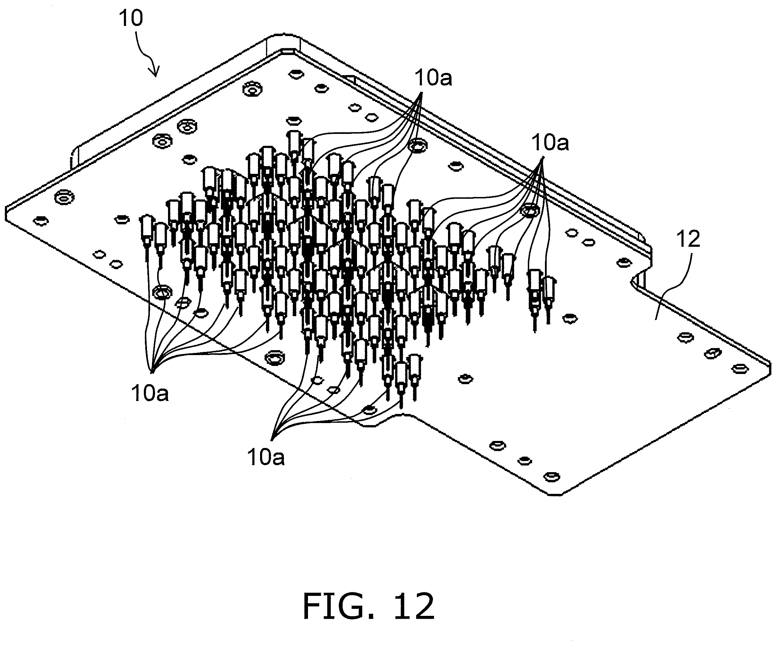

The probes 10a inserted into the through-holes 29b are held by the probe holder 10, which is made of resin, for example. As shown in Fig. 12, the probe holder 10 is integrated with a metal plate 12 and in this state is provided so that the plurality of probes 10a protrude from the metal plate 12.

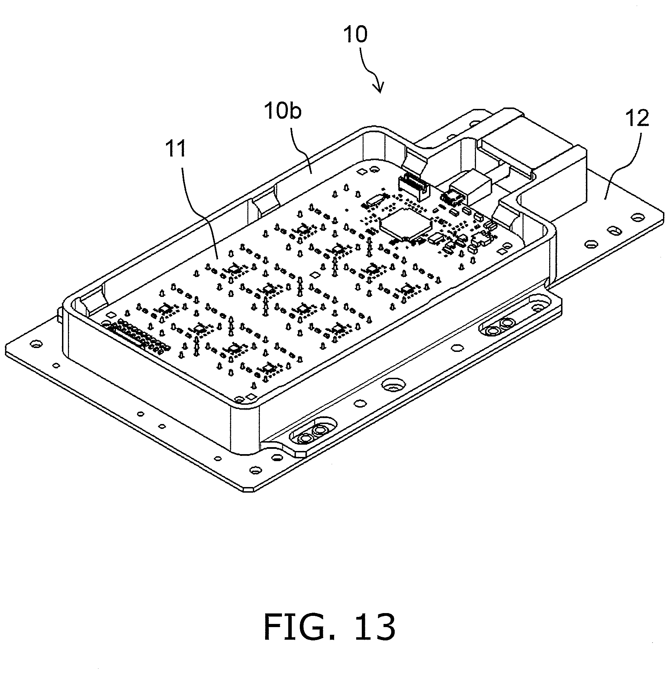

As shown in Fig. 13, the probe holder 10 has a metal plate 12 attached to its lower surface, and has on its upper surface a box-shaped probe box 10b that is open at the top.

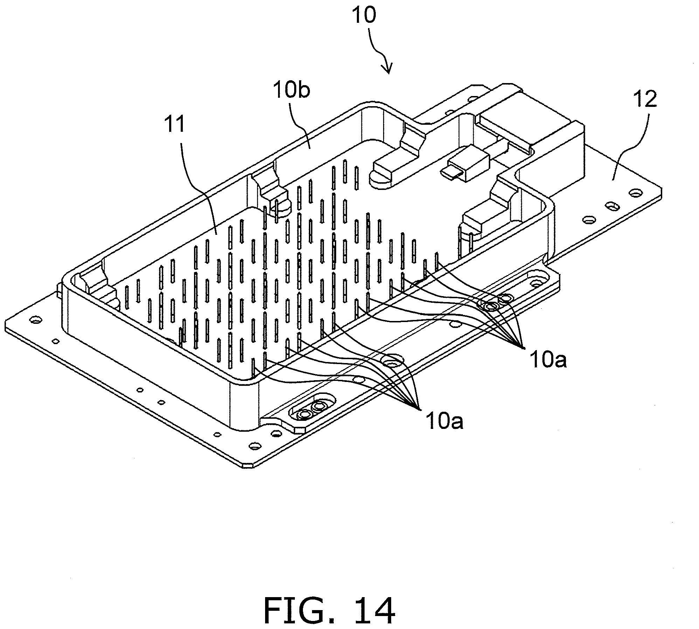

A board 11 to which the plurality of probes 10a are soldered is disposed on the bottom surface of the probe box 10b. That is, the probes 10a protrude from the bottom surface of the probe box 10b as shown in Fig. 14, and are electrically connected to the board 11 by being soldered in a state in which the board 11 is set on the bottom surface of the probe box 10b (see the portions in dashed circles in Fig. 16).

The upper and lower surfaces of the board 11 may be molded from resin to make them resistant to water and moisture (see the dots portions in Fig. 16).

When the probe holder 10 is placed on the upper surface of the top plate 29 in a state in which the probes 10a have been soldered to the board 11, the distal ends of the probes 10a come into contact with the electrodes 31c included in the connection terminal parts 31b of the sensors 30, as shown in Fig. 15.

That is, when the probe holder 10 holding the probes 10a in a state of protruding downward is disposed on the upper surface of the top plate 29, as shown in Fig. 16, the probes 10a are inserted into the through-holes 29b in the top plate 29 and make contact with the electrodes 31c constituting the connection terminal parts 31b of the sensors 30 disposed at the bottom portions of the through-holes 29b.

The probes 10a are biased downward by a spring (not shown), and move upward upon contact with the electrodes 31c, so their contact state with the electrodes 31c is maintained.

As shown in Fig. 16, a specific space S1 is formed between the upper surface of the top plate 29 and the lower surface (metal plate 12) of the probe holder 10. The top plate 29 is provided with the through-holes 29b that connect the specific space S1 side with the bottom plate 27 side. Consequently, the connection terminal parts 31b of the sensors 30 are exposed in the specific space S1 on the bottom plate 27 via the through-holes 29b.

Only the probes 10a protruding downward from the probe holder 10 are in the space S1 formed between the upper surface of the top plate 29 and the lower surface of the probe holder 10.

Here, the space S1 is formed so that its height is greater than the thickness of the top plate 29, for example.

Consequently, open spaces S1 are formed above the electrodes 31c constituting the connection terminal parts 3 1b to which voltage is applied via the probes 10a.

This means that even if condensation should occur on the surface of the sensor-equipped culture module 20 when the module is moved from a room temperature environment to the culture incubator 3 having an environment of high temperature and humidity, air permeability can be ensured by the open spaces S1. Accordingly, the vicinity of the connection terminal part 31b more readily adapts to the environment of high temperature and humidity, good ventilation makes it less likely that the condensed moisture will grow, and therefore it is less likely that adjacent electrodes 31c will be electrically connected by condensation.

As a result, leakage current is less likely to be caused by condensation that occurs when the sensor-equipped culture module 20 is moved from a room temperature environment to an environment of high temperature and high humidity, so more accurate measurement can be performed.

Also, with the configuration of this embodiment, the probe holder 10 has small diameter portions 10c, which have a smaller outside diameter than the upper portions, at the portions where the vicinity of the distal ends of the multiple probes 10a are held.

This minimizes the volume of the distal end portion of the probe holder 10 in the spaces S1 above the electrodes 31c constituting the connection terminal parts 31b with which the distal ends of the probes 10a make contact, and ensures the spaces S1 can be as large as possible.

This prevents the growth of moisture condensed around the electrodes 31c constituting the connection terminal parts 31b of the sensors 30, and effectively suppresses the occurrence of leakage current.

Furthermore, in this embodiment, the through-holes 29b in the top plate 29 into which the probes 10a are inserted are formed so as to communicate with the space above two adjacent electrodes 31c. That is, the through-holes 29b are formed so that one hole covers the range of two of the electrodes 31c.

Consequently, the spaces S1 formed above the electrodes 31c are opened up wider, making it possible to effectively suppress the growth of moisture condensed on the electrode surface where the electrodes 31c are disposed.

As shown in Fig. 17A, the electrodes 31c constituting the connection terminal parts 31b of the sensors 30 are provided such that the distance L between the electrodes 31c is greater than the width W of each electrode 31c.

This increases the distance between the electrodes 31c and makes it less likely that a water film formed by condensed moisture will electrically connect the electrodes 31c, which prevents leakage current from flowing between the electrodes 31c.

Furthermore, a water-repellent resist (water-repellent layer) 35 is provided on the surface of the base material 34 (PET sheet) of the sensors 30.

As shown in Fig. 17B, the moisture condensed on the electrode surfaces of the sensors 30 turns into spherical water droplets W1 due to the water repellency of the water-repellent resist 35. This effectively prevents the water droplets W1 from joining together between the electrodes 31c and forming a water film, which would otherwise allow leakage current to flow.

Furthermore, the water-repellent resist 35 is formed so that its thickness is greater than the thickness of the electrodes 31c.

This extends the creepage distance between the electrodes 31c and more effectively suppresses the occurrence of leakage current between the electrodes 31c.

Also, since the surfaces of the electrodes 31c are located lower than the water-repellent resist 35, it is less likely that the moisture condensed on the surface of the electrodes 31c will join up with the moisture condensed on the surface of the water-repellent resist 35.

Furthermore, the water-repellent resist 35 is provided with convex portions 35a that protrude upward at the edge portions adjacent to the outer periphery of the electrodes 31c.

Consequently, moisture condensed on the surface of the water-repellent resist 35 collects on the surface of the water-repellent resist, making it less likely that the moisture will join up on the electrode 31c side.

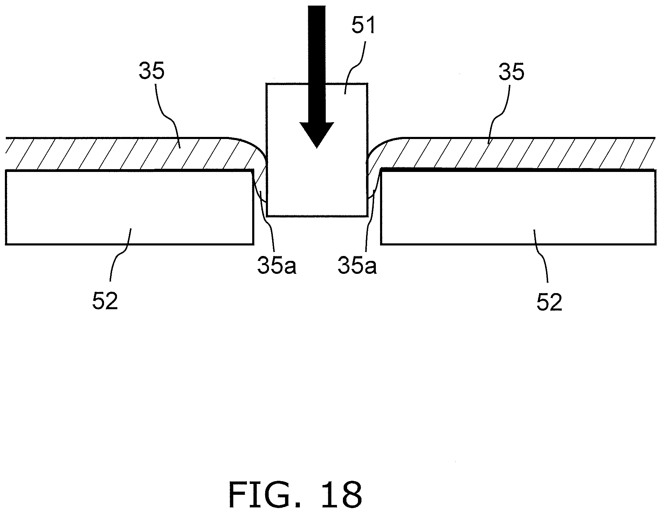

The convex portions 35a formed at the edge portions of the water-repellent resist 35 may be formed, for example, as shown in Fig. 18, by making use of a step formed in a cutout cross section when the water-repellent resist 35 formed on a base 52 is punched out using a pinnacle (common name) 51.

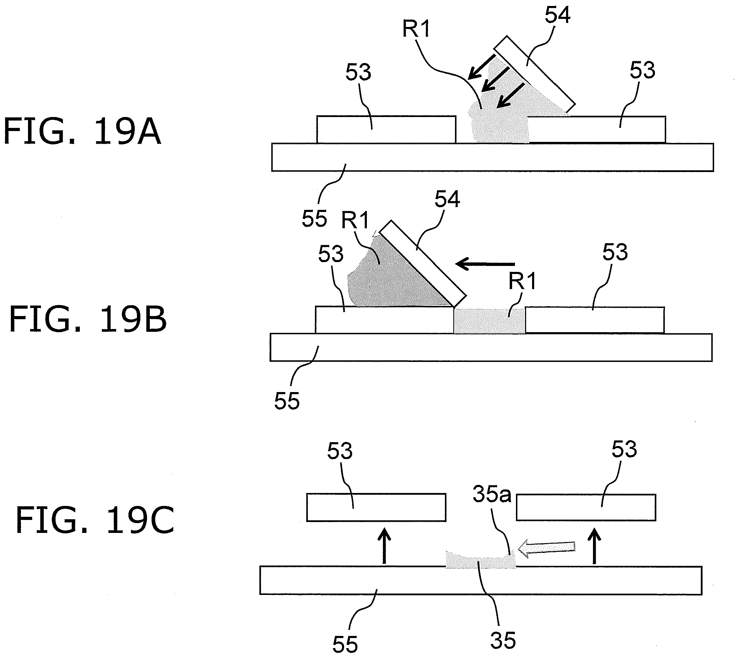

Alternatively, the convex portions 35a of the water-repellent resist 35 may be formed by screen printing while pressing a plate 54 against the upper surface of a form 53 as shown in Fig. 19B in a state in which a resist material R1 has been put into the form 53 placed on a base 55 as shown in Fig. 19A.

In this case, as shown in Fig. 19B, when the form 53 is lifted up from the base 55, the protruding portion formed at the end of the water-repellent resist 35 due to the saddle phenomenon may be used as the convex portion 35a.

The thicker is the water-repellent resist 35, the more the ends of the protruding portion will naturally rise up due to the saddle phenomenon, so these raised portions can be utilized as the convex portions 35a.

### Main Features

In order to suppress the occurrence of leakage current due to condensation and perform analysis properly, the sensor unit 28 of this embodiment includes the sensors 30, the bottom plate 27, the top plate 29, and the probe holder 10, as shown in Fig. 16. The sensors 30 have the connection terminal parts 31b including a plurality of electrodes 31c that are electrically connected to the sensing parts 31a and to which a specific voltage is applied when measuring a component of the culture medium X. The bottom plate 27 has an upper surface to which the connection terminal parts 31b are attached and a lower surface that is on the opposite side form the upper surface. The top plate 29 has the through-holes 29b into which are inserted the probes 10a that make contact with the electrodes 31c and apply a specific voltage, and is disposed so as to sandwich the connection terminal parts 31b along with the bottom plate 27. The probe holder 10 holds the probes 10a so that the probes 10a protrude toward the connection terminal parts 31b, has the metal plate 12 disposed opposite the connection terminal parts 31b of the sensors 30, and is disposed so that the specific spaces S 1 are formed between the lower surface of the metal plate 12 and the top plate 29.

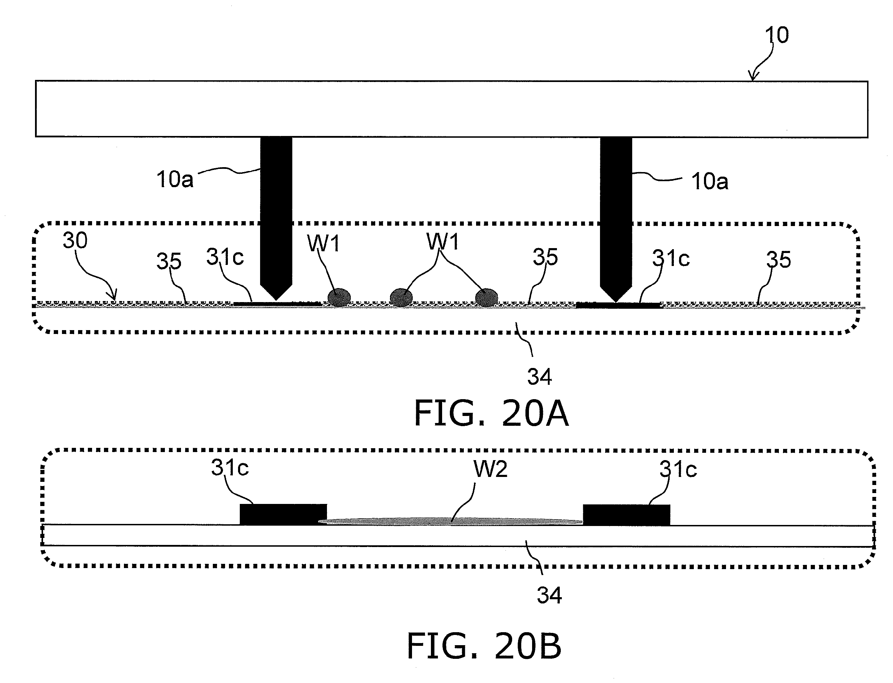

Consequently, condensation occurs on the surface of the sensor unit 28 that has been placed in a room temperature environment or an environment of high temperature and humidity, but the specific spaces S1 are formed at the portions opposite the connection terminal parts 31b including the plurality of electrodes 31c. Accordingly, as shown in Fig. 20A, it is less likely that the moisture (water droplets W1) condensed on the surface of the sensor 30 will grow to the point of electrically connecting the electrodes 31c.

Consequently, even when the device is used in an environment of high temperature and high humidity, as shown in Fig. 20B, the moisture condensed between the electrodes 31c forms a water film W2, which effectively suppresses leakage current, and this allows various kinds of measurement related to cell culture analysis to be performed very accurately.

The main components of the sensor unit 28 in this embodiment will now be described in further detail.

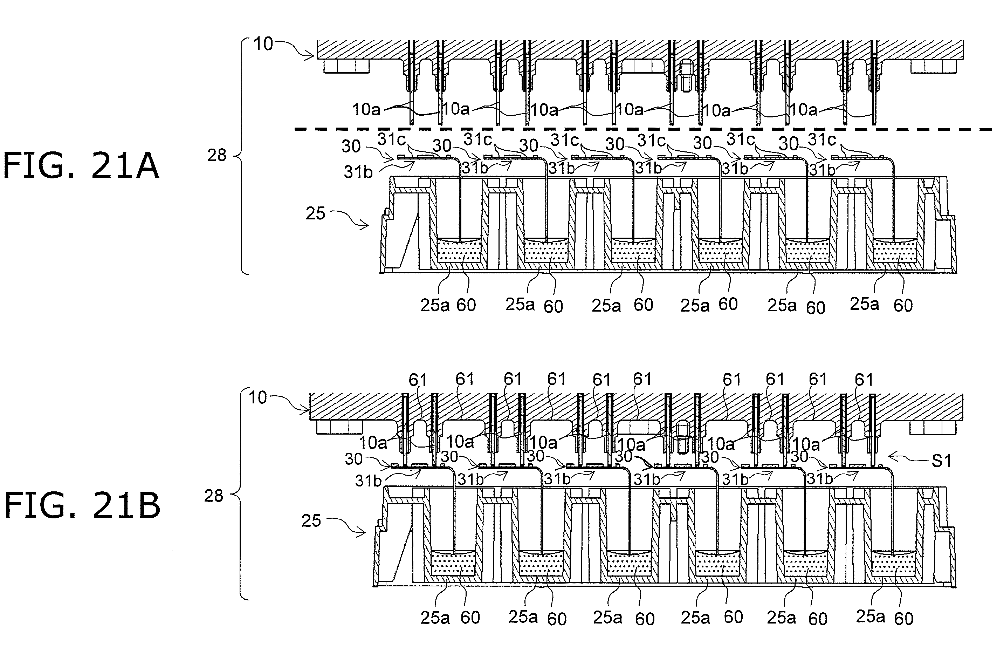

Fig. 21A is a cross-sectional view of the sensor unit 28 immediately before used sensors 30 are replaced.

The sensor unit 28 of this embodiment is a device constituting part of the cell culture analyzing device 1 that is disposed inside the culture incubator 3. The sensor unit 28 is configured to include a disposable part (the members below the dashed line in Fig. 21A) and a reusable part (the members above the dashed line in Fig. 21A).

The cell culture analyzing device 1 estimates the cell culture state by measuring metabolic components of the culture medium with the sensor unit 28 while culturing the target cells in a culture medium in a plurality of wells (culture containers) 25a in the culture incubator 3. When replacing the target cells, the wells (culture containers) 25a and the sensors 30 of the sensor unit 28 are the primary portions that are replaced, and the other connection parts electrically connected to the sensors 30 (the members above the dashed line in the drawing) are reused.

The disposable part (below the dashed line in the drawing) mainly includes the sensors 30, the culture medium 60 (as a liquid sample) in which the sensing parts 31a of the sensors 30 are immersed for measurement, and the wells (culture containers) 25a for holding this culture medium.

The reusable portion (above the dashed line in the drawing) mainly includes the probes (connection parts) 10a that directly make contact with the electrodes 31c of the connection terminal parts 31b of the sensors 30, and the probe holder (connection part holder) 10.

Here, the disposable part and the reusable part are configured to be detachable, and the user replaces the disposable part inside the culture incubator 3.

As discussed above, the inside of the culture incubator 3 is an environment of high temperature and high humidity (37°C, humidity 90%). In replacing the disposable part in such an environment of high temperature and high humidity, the disposable part is stored in a refrigerator or at room temperature immediately before use. Therefore, in the hot and humid environment of the culture incubator 3, condensation is likely to occur on the disposable part due to the temperature difference between the inside of the culture incubator 3 and the disposable part.

Also, if the reusable part is not allowed to stand long enough to reach the internal temperature after being installed in the culture incubator 3, there is a risk of condensation forming on the surface.

In particular, the probes 10a included in the reusable part are made of a conductive metal, have a large thermal capacity, and take time to reach the temperature inside the culture incubator 3, and as such are more susceptible to the adhesion of moisture. Therefore, if the reusable part is used in the culture incubator 3 without allowing it to stand for a sufficient time after being installed, the probes 10a on which a large amount of condensation has occurred will be electrically connected to the electrodes 31c whose temperature is still close to room temperature.

As a result, after the disposable part has been replaced, moisture adhering to the reusable part and condensation caused by temperature changes in the disposable part may cause a short circuit or leakage current between electrical contacts.

If the electrical contacts between the connection terminal parts 31b of the sensors 30 and the probes 10a that make contact with the connection terminal parts 3 1b are sealed in a waterproof structure, then any moisture adhering to the reusable part will be sealed in the waterproof structure at the time of replacement. This ends up making it more likely that a short circuit or leakage current will occur between the electrical contacts as a result of the sealed-in moisture.

In view of this, in the sensor unit 28 of this embodiment, the connection terminal parts 31b of the sensors 30, which are electrical contact portions, and the area around the probes 10a that make contact with the connection terminal parts 3 1b are more open to the atmosphere of the culture incubator 3. As a result, even if condensation should occur, the moisture that is generated at that time can be dried by the air current circulating within the culture incubator 3.

Fig. 21B is a cross-sectional view of the sensor unit 28 after the sensors 30 have been replaced.

As shown in Fig. 21B, the sensor unit 28 measures a component in the culture medium 60 in a state in which the lower ends of the sensors 30 are immersed in the culture medium 60 (serving as a liquid sample) contained in the wells (culture containers) 25a.

The configuration of the disposable part of the sensor unit 28 will now be described.

As shown in Fig. 8, a sensor 30 has a main body 31, sensing parts 31a that are disposed on the main body 31 and immersed in the culture medium 60, and connection terminal parts 31b that are electrically connected to the sensing parts 31a and include a plurality of electrodes 31c (see Fig. 11) to which a specific voltage is applied when measuring a component of the culture medium 60.

Next, the configuration of the reusable portion of the sensor unit 28 will be described.

The probes 10a apply a specific voltage to the connection terminal parts 31b of the sensor 30 while in contact with the connection terminal parts 31b.

The probe holder 10 holds the upper portions of the probes 10a so that the probes 10a protrude toward the connection terminal parts 31b of the sensor 30.

The probe holder 10 also has an opposing surface 61 disposed opposite the connection terminal parts 31b of the sensor 30. Consequently, spaces S1 are formed between the opposing surface 61 and the connection terminal parts 31b of the sensor 30, which communicate with the spaces faced by the adjacent electrodes 31c of the connection terminal parts 31b of the sensor 30.

As discussed above, the probe holder 10 holds the probes 10a so that the probes 10a protrude toward the connection terminal parts 31b. Consequently, a specific height is formed between the opposing surface 61 and the connection terminal parts 31b of the sensor 30, and the spaces S1 become non-capillary spaces in which capillary action does not work on the liquid near the spaces S1.

Since the spaces S1 are non-capillary spaces, moisture is not absorbed between the opposing surface 61 and the connection terminal parts 31b of the sensor 30. This prevents excess moisture from entering around the connection terminal parts 31b through capillary action.

In the cell culture analyzing device 1, the spaces S1 are configured to be spaces that are open to the outside of the device in a substantially horizontal direction. This causes the circulating air current inside the culture incubator 3 to be generated in the spaces S1 as well. Consequently, the spaces S1 connect the spaces faced by the adjacent electrodes 31c of the connection terminal parts 31b of the sensor 30 in a substantially horizontal direction, so any moisture produced around the electrodes 31c can be evaporated and dried. As a result, short circuits between electrical contacts and leakage current can be effectively prevented.

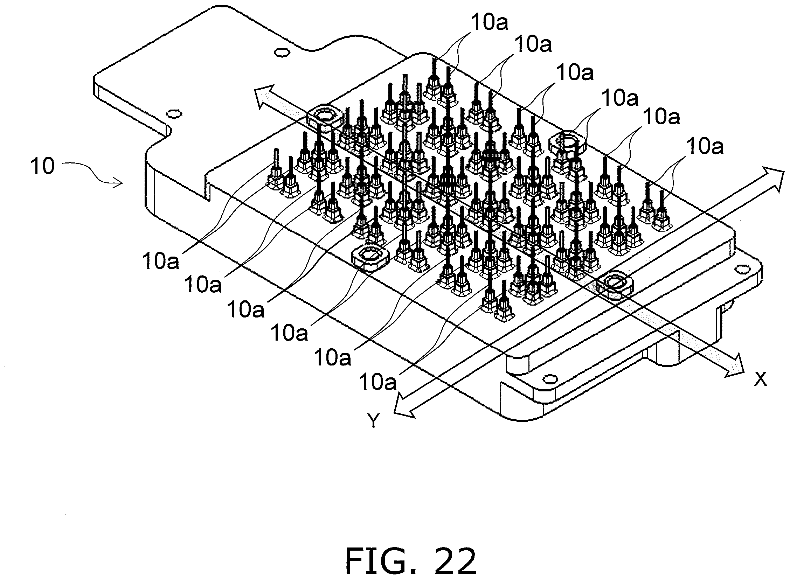

Fig. 22 is an oblique view of the probes 10a and the probe holder 10, in a state in which the probes 10a are facing upward.

Here, the connection terminal parts 31b on the sensor 30 side where the probes 10a make contact is provided with four electrodes 31c for each sensor 30.

Similarly, as shown in Fig. 22, the probes 10a each have four electrodes, and are arranged in four rows in the X direction and six in the Y direction.

The adjacent probes 10a are provided in spaces that communicate with each other. Also, the probes 10a are provided in spaces that are open to the outside of the device so that circulating air can easily flow in the directions indicated by the arrows in the X and Y directions in Fig. 22 (that is, the substantially horizontal direction of the device when in use state).

As a result, in the spaces S 1, the spaces facing the adjacent electrodes 31c included in the connection terminal parts 31b of the sensor 30, and the adjacent probes 10a of the probe holder 10 communicate with each other. Consequently, any moisture generated around the electrodes 31c and the corresponding probes 10a can be evaporated and dried, and the occurrence of short circuits and leakage current between electrical contacts can be effectively prevented.

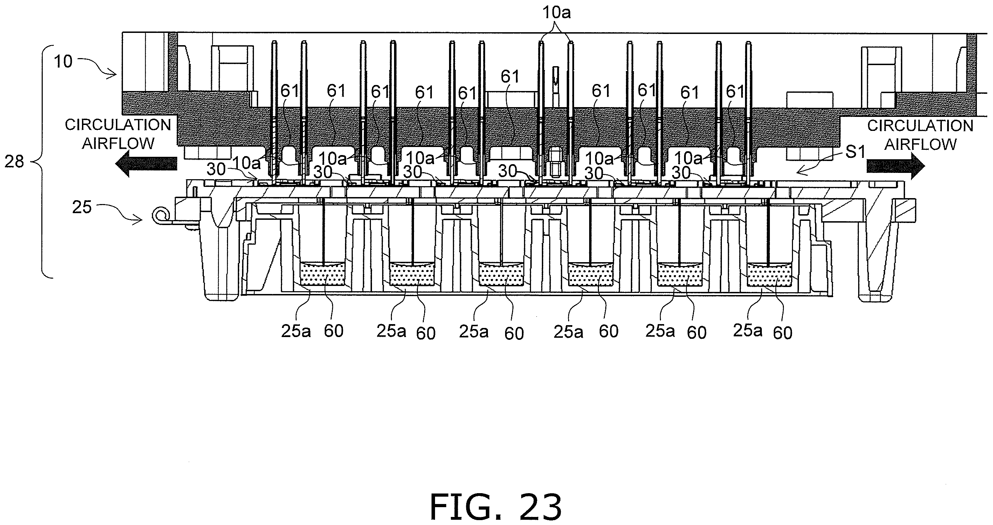

Fig. 23 is a cross-sectional view of the state when the sensors 30 and the probe holder 10 have been set on the well plate 25.

As shown in Fig. 23, the probes 10a can be directly seen from the side of the device, that is, the spaces S1 form a passageway for the circulating air current in the culture incubator 3 because they are spaces that are open in the substantially horizontal direction of the device when in use state.

Furthermore, any moisture generated around the electrodes 31c and the corresponding probes 10a provided in the spaces communicating within the spaces S1 is effectively evaporated and dried by the circulating airflow. As a result, short circuits and leakage current between electrical contacts can be effectively prevented.

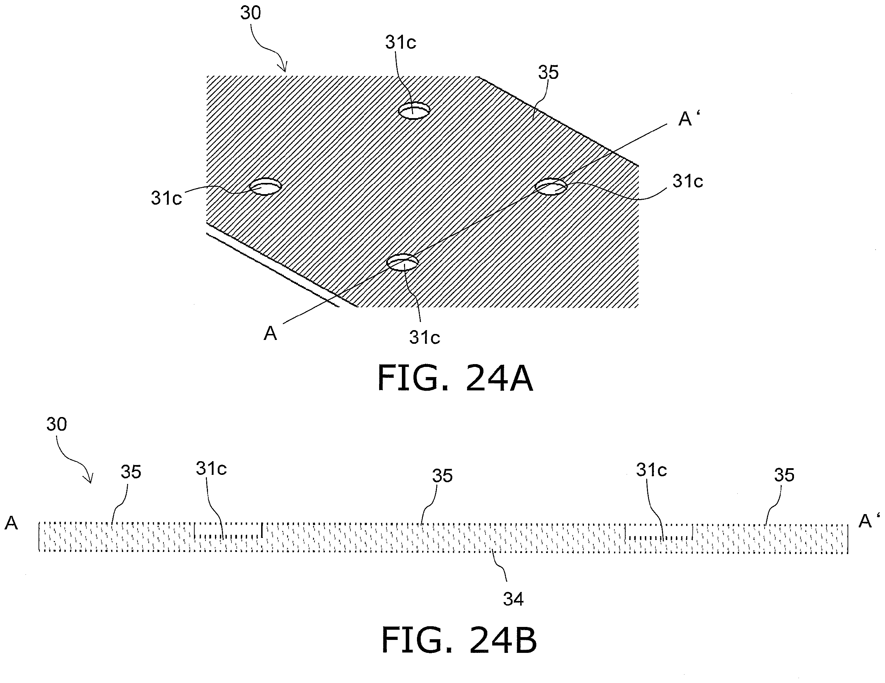

Fig. 24A is an oblique view of a connection terminal part 31b of a sensor 30.

Fig. 24B is a cross-sectional view of the connection terminal part 31b along the A-A' line in Fig. 24A.

As shown in Figs. 24A and 24B, the electrodes 31c included in the connection terminal part 31b are disposed so that the distance between adjacent electrodes 31c is greater than the width of the electrodes 31c.

Also, the water-repellent resist 35 is located between the electrodes 31c, and the electrode 31c portion is located lower than the portion of the water-repellent resist 35 between the electrodes 31c. Consequently, the electrodes 31c are disposed in recesses formed in the base material 34, and the portion of the water-repellent resist 35 between the electrodes 31c is formed as a convex portion of the base material 34.

That is, the length of the water-repellent resist 35 in the substantially horizontal direction is greater than the length of the electrode 31c in the substantially horizontal direction, and the thickness of the water-repellent resist 35 is greater than the thickness of the electrodes 31c.

This causes any moisture generated by condensation to accumulate in the recesses of the base material 34 in which the electrodes 31c are provided, effectively preventing the occurrence of short circuits or leakage current between the electrodes 31c. Also, since the length of the water-repellent resist 35 in the substantially horizontal direction is greater than the length of the electrodes 31c in the substantially horizontal direction, a sufficient distance is ensured between the electrodes 31c, effectively preventing the occurrence of short circuits or leakage current between the electrodes 31c.

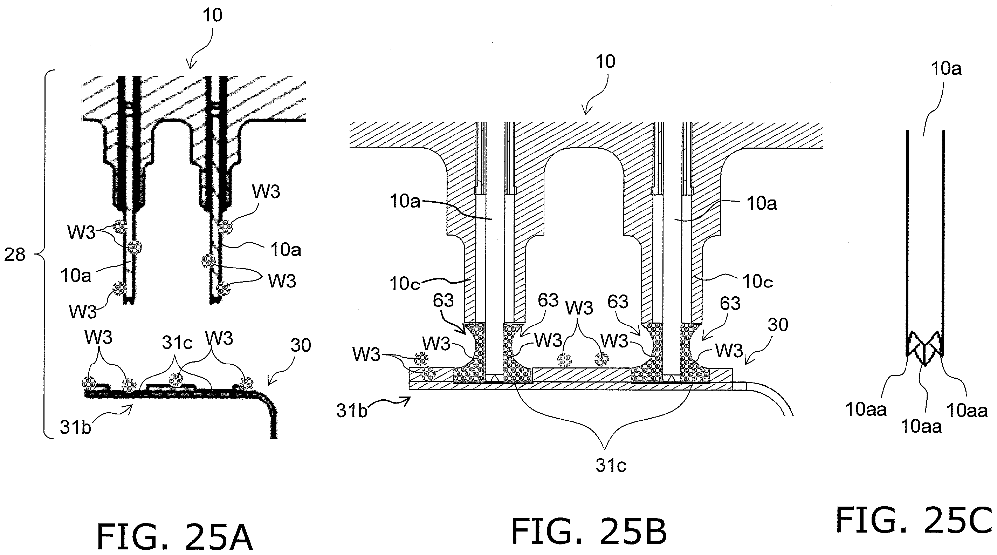

Fig. 25A shows a detail cross-sectional view of the sensor unit 28 immediately before replacement of the used sensors 30.

Sodium, potassium, chlorine, and the like adhere to the probes 10a over long-term use. Therefore, condensed water W3, in which an electrolyte component is dissolved and through which leakage current easily flows, clings to the probes 10a. This condensed water W3 also clings to the connection terminal parts 31b of the sensor 30 below.

Here, the probes 10a are biased downward in the drawing by a spring or other such elastic member, and upon making contact with the electrodes 31c, slide inside the probe holder 10. Consequently, even if the positions of the multiple electrodes 31c vary in the height direction, the probes 10a can absorb this positional variance, allowing a stable electrical connection to be achieved.

On the other hand, when the probes 10a and the electrodes 31c are electrically connected, the probes 10a slide into the small diameter portion 10c of the probe holder 10, so that the condensed water W3 containing electrolyte component clinging to the probes 10a is scraped off by the lower end of the small diameter portion 10c in the direction of the electrodes 31c.

Therefore, as shown in Fig. 25B, in a state in which the probes 10a and the electrodes 31c are electrically connected (in the sensor unit 28 after the sensors 30 have been replaced), the condensation water droplets W3 adhering to the probes 10a are scraped off by the lower end of the small diameter portion 10c, and condense on the electrodes 31c into droplets.

As shown in Fig. 25B, the diameter of the distal end of the probe 10a that makes contact with the electrode 31c is smaller than the size of the electrode 31c in the radial direction.

Consequently, the condensed water W3 produced by the above-mentioned sliding action can be held within the recesses in which the electrodes 31c are provided, which effectively prevents the occurrence of short circuits or leakage current between the electrodes 31c due to condensation.

Also, as discussed above, the probe holder 10 has the small diameter portion 10c, whose outside diameter where the probes 10a are held is smaller than the upper end (second end) side on the opposite side from the lower end, at the lower end (first end) on the electrode 31c side in the portion for holding the probes 10a.

This allows a gap 63 to be formed between the small diameter portion 10c and the electrodes 31c. As a result, even if there is a large amount of condensed water W3, a large amount of water can be held in the gap 63 by the strong adsorptive force of the meniscus (liquid bridge) generated in the gap 63. This effectively prevents the occurrence of short circuits or leakage current between the electrodes 31c.

Fig. 25C is a detail view of the distal end of a probe 10a.

The probe 10a has a crown shape including a plurality of sharp protrusions 10aa at its distal end. Consequently, the sharp protrusions 10aa penetrate the oxide film formed on the surface of the electrode 31c or the probe 10a, which ensures electrical connection between the electrode 31c and the probe 10a.

Furthermore, the occurrence of poor electrical connection, which can happen when dust (loose fibers) adhering to the probes 10a through long-term use becomes embedded, can be effectively prevented, for example.

Also, even if the electrodes 31c are made of a material that is easily transferred, such as carbon or solder, the self-cleaning effect will prevent the accumulation of such materials on the distal ends of the probes 10a.

Here, the probes 10a are preferably made of a highly corrosion-resistant material such as an SUS alloy, a palladium alloy, palladium, rhodium, iridium, tungsten, or titanium, all of which are resistant to corrosion by moisture.

This allows highly reliable electrical connection to be performed even when the electrical connection is made in an environment of high temperature and high humidity inside the culture incubator 3 where poor contacts may result.

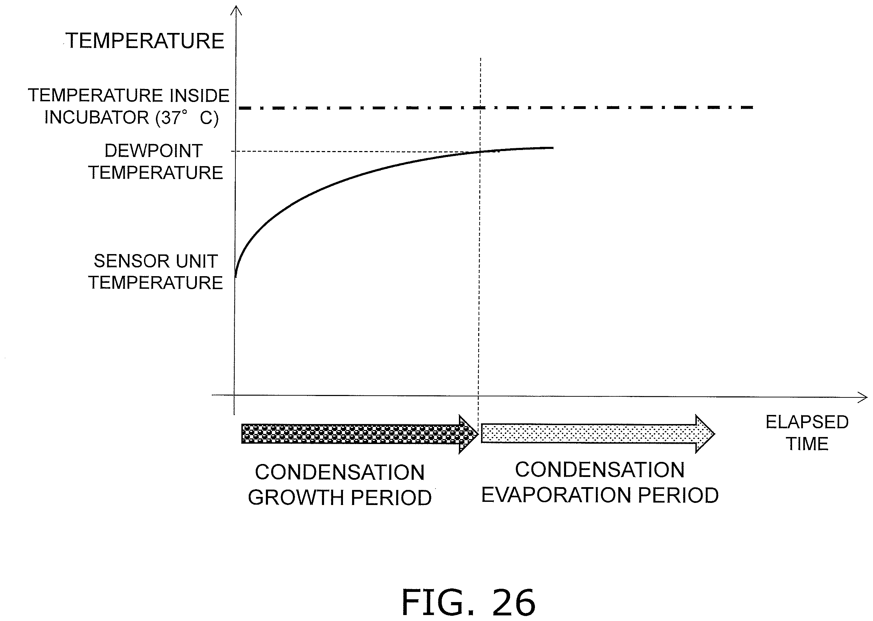

Fig. 26 is a graph of the relation between the elapsed time during replacement of the disposable sensors 30 and the temperature of the disposable parts including the sensors 30.

After the sensors 30 are replaced with new ones, condensation occurs on the surface of the disposable part including the new sensors 30 while the temperature of this part gradually approaches the temperature inside the culture incubator 3, which is a high-temperature and high-humidity environment, until the dewpoint temperature is finally reached. During this process, as discussed above, moisture such as the condensed water W3 is actively drawn into the recesses in which the electrodes 31c of the connection terminal parts 31b are provided.

Then, after the disposable part including the sensors 30 has reached the temperature inside the culture incubator 3, as mentioned above, the moisture in the spaces S1 open to the outside is evaporated and dried by the circulating airflow that is formed in the culture incubator 3 in a substantially horizontal direction.

Consequently, moisture such as the condensation water W3 generated in the spaces S 1 grows up until the sensor unit 28 reaches the dewpoint temperature, but once the dewpoint temperature is reached, the circulating airflow generated within the culture incubator 3 can evaporate and dry the generated condensation water, etc.

As a result, the occurrence of short circuits or leakage current between the electrodes 31c disposed in the spaces S1 can be effectively prevented.

As described above, with the sensor unit 28 in this embodiment, after the sensors 30 have been replaced, the above-mentioned two-stage technique (the holding of the condensation water W3, and its evaporation and drying) can effectively prevent the occurrence of short circuits or leakage current between the electrodes 31c.

### Other Embodiments

An embodiment of the present invention was described above, but the present invention is not limited to or by the above embodiment, and various modifications are possible without departing from the gist of the invention.
(A) In the above embodiment, an example was given in which the water-repellent resist 35 was formed on the electrode surface on which the plurality of electrodes 31c constituting the connection terminal parts 31b of the sensors 30 were arranged. However, the present invention is not limited to this.

For example, the configuration may be such that the electrode surface on which the connection terminal parts are provided has no water-repellent layer.

Nevertheless, as described in the above embodiment, a configuration in which a water-repellent layer is provided on the electrode surface is preferable because condensed moisture will be less prone to changing into droplets and forming a water film.

### (B)

In the above embodiment, an example was given in which the through-holes 29b in the top plate 29 into which the probe 10a was inserted were formed so as to communicate with the space above two adjacent electrodes 31c. However, the present invention is not limited to this.

For example, the through-holes in the top plate may be provided one for each electrode, or one for three or more electrodes.

### (C)

In the above embodiment, an example was given in which the upper ends of four sensors 30 were linked by a linking portion 33 so that the four sensors 30 formed a set. However, the present invention is not limited to this.

For example, the number of sensors linked by the linking portion may be three or fewer, or may be five or more.

In any case, the positions of the mutually linked sensors will be accurately defined, so the positional accuracy of the sensors can be improved.

### (D)

In the above embodiment, an example was given in which the sensors 30 were used in a state of having been bent at the bent portions 32. However, the present invention is not limited to this.

For example, the configuration may be such that the sensors are used without being bent.

Here again, configuring the sensors to be linked by linking portions at the upper end portions of the main bodies of the sensors provides the same effect as above, that of improving the positional accuracy of the sensors.

### (E)

In the above embodiment, an example was given in which the sensors 30 had a substantially inverted T shape, but the present invention is not limited to this.

For example, a sensor that is substantially I-shaped or substantially L-shape may be used instead.

### (F)

In the above embodiment, an example was given in which the sensor-equipped culture module 20 placed in a room temperature (25°C) environment was put under an environment of high temperature and high humidity (37°C, a humidity of at least 90%) in the culture incubator 3. However, the present invention is not limited to this.

For example, the environment to which the sensor module is placed before being put in a high-temperature environment is not limited to one of room temperature, and may be any environment suitable for a pre-culture treatment of the sensor module.

Alternatively, the same effect as above can be obtained when a sensor module that has been pretreated at a temperature lower than room temperature is placed in an environment of room temperature, or of a temperature and humidity higher than room temperature.

### INDUSTRIAL APPLICABILITY

The sensor unit of the present invention exhibits the effect that the occurrence of leakage current attributable to condensation can be effectively suppressed even when used in an environment of high temperature and high humidity, and is therefore widely applicable to a variety of sensor units used in cell culture analysis.

### REFERENCE SIGNS LIST

1 cell culture analyzing device
2 analysis unit
3 culture incubator
3a door
4 control unit
5 electrical cable
10 probe holder (connection part holder)
10a probe (connection part)
10aa protrusion
10b probe box
10c small diameter part
11 board
12 metal plate
20 sensor-equipped culture module
21 main body
22 drawer
23 lifting mechanism
23a mounting base
23b, 23c, 23d, 23e arm
23ba rotation center
24 adaptor bottom
24a hinge portion
25 well plate
25a well (culture container)
26 adaptor top
27 bottom plate
27a through-hole
27b support portion
28 sensor unit
29 top plate
29a pressing portion
29b through-hole
30 sensor
31 main body
31a sensing part
31b connection terminal part
31c electrode
32 bent portion
33 connection part
34 base material (PET sheet)
35 water-repellent resist (water-repellent layer)
35a convex portion
40 leg
41a through-hole
41b through-hole
51 pinnacle
52 base
53 form
54 plate
55 base
60 culture medium
61 opposing surface
62 gap
63 gap
C1 safety cabinet
L distance
R1 resist material
S1 space
W width
W1 water droplet
W2 water film
W3 condensation water
X culture medium

## Claims

1. A sensor unit for measuring components of a liquid sample placed in a culture container, the sensor unit comprising:
a sensor having a main body, a sensing part that is disposed on the main body and is immersed in the liquid sample, and a connection terminal part including a plurality of electrodes that are electrically connected to the sensing part and to which a specific voltage is applied when the components of the liquid sample are measured;
a connection part configured to make a contact with the electrodes of the connection terminal part of the sensor and apply a specific voltage; and
a connection part holder configured to hold the connection part so that the connection part protrudes toward the connection terminal part of the sensor, has an opposing surface that is disposed opposite the connection terminal part of the sensor, and forms a non-capillary space between the opposing surface and the connection terminal part of the sensor to allow communication with a space facing the adjacent electrodes of the connection terminal part of the sensor.

2. The sensor unit according to claim 1,
wherein the connection part holder has a small diameter portion, whose outside diameter where the connection part is held is smaller than a second end portion on an opposite side of a first end portion, at the first end portion on a side of the electrodes of a portion where the connection part is held.

3. The sensor unit according to claim 2,
wherein a plurality of the small diameter portions are provided in a one-to-one with respect to the plurality of connection parts.

4. The sensor unit according to claim 1,
wherein the non-capillary space is a space that is open to the outside in a substantially horizontal direction when in use state.

5. The sensor unit according to claim 1,
wherein the sensor is a disposable member,
the connection part and the connection part holder are reusable members, and
the sensor is configured to be detachable from the connection part and the connection part holder.

6. The sensor unit according to claim 1,
wherein the plurality of electrodes included in the connection terminal part are disposed so that a gap between adjacent electrodes is greater than a width of the electrodes.

7. The sensor unit according to claim 1,
wherein the connection part has a crown shape including a plurality of protrusions at a distal end.

8. The sensor unit according to claim 1,
wherein a material of the connection part includes any of an SUS alloy, a palladium alloy, palladium, rhodium, iridium, tungsten, and titanium.

9. The sensor unit according to claim 1,
further comprising a water-repellent layer that is formed on an electrode surface on which the electrodes of the sensor are disposed, and that is water repellent.

10. The sensor unit according to claim 9,
wherein a thickness of the water-repellent layer is greater than a thickness of the electrodes.

11. The sensor unit according to claim 10,
wherein the plurality of electrodes are provided in recesses formed by a difference in thickness between the water-repellent layer and the electrodes.

12. The sensor unit according to claim 10 or 11,
wherein the water-repellent layer has a convex portion protruding in a direction away from the electrode, at an edge portion adjacent to the electrodes.

13. The sensor unit according to claim 10 or 11,
wherein a diameter of the connection part that makes contact with the plurality of electrodes is smaller than a diameter of the electrodes.

14. The sensor unit according to claim 1, further comprising:
a linking portion configured to link the plurality of sensors on an upper end side of the main body; and
a bent portion where the plurality of sensors are bent such that the linking portion is bent relative to the main body along a bending line that is substantially parallel to a lengthwise direction of the linking portion.

15. A cell culture analyzing device, comprising
the sensor unit according to claim 1.
